# EUROPEAN PATENT SPECIFICATION

(11) **EP 1 039 904 B1**
(45) Date of publication and mention of the grant of the patent: **28.05.2003**
(21) Application number: 98960655.3
(22) Date of filing: 03.12.1998
(51) Int. Cl.: A61K 31/415, A61K 31/40, A61P 31/00

(54) **SYNERGISTIC ANTIMICROBIAL COMPOSITIONS**
SYNERGISTISCHE ANTIMIKROBIELLE ZUSAMMENSETZUNGEN
COMPOSITIONS ANTIMICROBIENNES SYNERGIQUES

(30) Priority: 03.12.1997 US 67429 P
(43) Date of publication of application: 04.10.2000
(73) Proprietor: Goodman, Thomas, C., Tucson, AZ 85718-1426 (US)
(72) Inventor: Goodman, Thomas, C., Tucson, AZ 85718-1426 (US)
(74) Representative: Bodemann, Doris
(86) International application number: US9825568
(87) International publication number: WO99027927

(56) References cited:
- CHEMICAL ABSTRACTS, Vol. 100, 1984, (Columbus, Ohio, USA), Abstract No. 188650, HAMILTON-MILLER J.M.T., "Antimicrobial Activity of 21 Antineoplastic Agents" XP002955365 & BR. J. CANCER, 1984, 49(3), 367-369
- CHEMICAL ABSTRACTS, Vol. 125, 1996, (Columbus, Ohio, USA), Abstract No. 33495, SKIBO E.B. et al., "The Organic Chemistry of the Pyrrolo(1,2-a)Benzimidazole Antitumor Agents. An Example of Rational Drug Design" XP002955366 & & SYNLETT, 1996, (4), 297-309

## Description

### TECHNICAL FIELD AND INDUSTRIAL APPLICABILITY OF INVENTION

This invention relates to novel antimicrobial compositions and their use in treating microbial disease

### BACKGROUND OF THE INVENTION

### Antibiotic Resistance

Overexposure to antibiotics is one factor which propels bacteria to develop diverse mechanisms of resistance so that the antibiotics are no longer effective. The growth of antibiotic resistant bacteria is becoming more problematic. This is especially true since the discovery of bacterial strains resistant to vancomycin, vancomycin-resistant enterococci (VREs), and organisms having intermediate sensitivity to vancomycin (VISA). For the last 30 years, vancomycin has been considered the last line of defense against bacterial infection.

Another potential cause for concern is the emergence of unusual patterns of secondary infection, i.e. super-infection. In super-infection, a secondary bacterial infection occurs while a patient is being treated with an antibiotic for another bacterial infection. The secondary infection may be treatable with known antibiotics or may be resistant to treatment.

Several strategies have been undertaken to combat antibiotic resistant bacteria. These strategies include the combination of known antibiotics, the chemical modification of existing antibiotics, and the development of brand new antibiotics. Combinations of antibiotics and compounds which inhibit the breakdown of the antibiotics by the resistant organism, such as described in U.S. Patent No. 4,145,430, (Hunt, E. ) have proven useful. In addition, binary antibiotics such as quinupristin/dalfopristin (Synercid(r), Rhone-Poulenc Rorer, College Park, PA) are known to act in concert by binding to different sites on the 50S bacterial ribosome so as to cause cell death (Jamjian, C. et al., Diagn. Microbiol. Infect. Dis. 27:129-38, 1997; Chant, C. et al., Ann. Pharmacother. 29:1022-27, 1995; Finch RG, Drugs 1996, 51(Suppl 1):31-37, 1996).

Efforts have also been made to try to reduce the toxicity of existing antibiotics (Song B-B, et al., JPET 282:369-77, 1997). An attempt also has been made to convert drug-resistant bacteria to drug sensitivity by the application of external polynucleotides, referred to as guide sequences (Guerrier-Takada C, et al., PNAS 94:9468-72, 1997). All of these efforts demonstrate the intense interest in developing novel strategies to combat both antibiotic resistant bacteria and super-infection.

### Mitomycin C

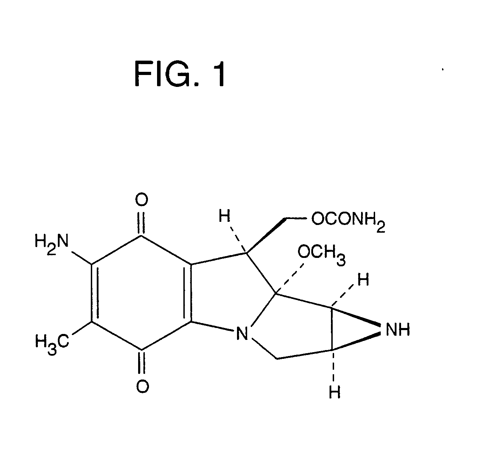

Mitomycin C is a powerful antitumor antibiotic. The structure of mitomycin C (I) is illustrated in Figure 1. Mitomycin C's mechanism of action is well known (Frank RW, "the Mitomycin Antibiotics," Fortschritte D Chem Org Naturst. 38:1-45, 1978). The first step involves the reduction of the quinone to the indole to form a reductively activated species. Then carbamate and aziridine groups from carbons C1 and C10, respectively, are eliminated to form the active quinone methide. The quinone methide is susceptible to nucleophilic attack by purines of complementary strands of DNA resulting in cross-linked DNA strands. The drug cross-links at 5' CpG sequences (Millard, JT et al., J. Am. Chem. Soc. 112:3637, 1990). Other mitomycins, such as mitomycin A and B, also function in a similar way.

Mitomycin C was first purified from Streptomyces caepitosus. Mitomycin C is presently purified from a number of Streptomyces bacteria. Alternatively, mitomycin C and its derivatives can be synthesized by chemical means.

Mitomycin C-producing bacteria are resistant to the deleterious effects of mitomycin C and potential self-destruction. The resistance-inducing mechanism has only recently begun to be understood. A protein (MRCA) that confers resistance to mitomycin C and its analogs is induced specifically by mitomycins, in particular mitomycins C and A (August PR, et al., Gene 175:261-67, 1996). The protein (MCRA) is related to the flavin adenine di-nucleotide dependent oxidoreductase family and acts by oxidizing mitomycins so that the mitomycins are no longer in their reductively active form. The enzyme can oxidize a variety of mitomycin analogs. A second protein (MRCB) is also induced by mitomycin C. MRCB may act in concert with MRCA or may be a transcriptional activator protein (August PR, et al., J. Bacteriol. 176(14): 4448-54, 1994).

Computer-assisted comparison of the MRCA sequence with other protein sequence databases has revealed significant similarity of MCRA with other oxidases, such as the amino terminal half of 6-hydroxy-D-nicotine oxidase from Arthrobacter oxidans. The enzyme operates in nicotine catabolism by catalyzing a two-electron oxidation of the D-hydroxynicotine pyrrolidine ring. Significant sequence similarity between MRCA and three oxygen oxido-reductases from bacterial, plant and animal systems has been identified.

### 6-acetamido-7-methyl-2,3-dihydro-1H-pyrrolo [1,2-a]benzimidazole-5.8-dione (APBI)

The pyrrolo[1,2-]benzimidazoles are members of a class of antitumor agents useful against a variety of cancer cell types (Islam I and Skibo EB, J. Org. Chem. 55:3195-3205, 1990). The compounds were synthesized to create mitomycin C analogs which are less cytotoxic than mitomycin C. The pyrrolo[1,2-]benzimidazoles are divided into several subclasses based on their mechanism of action. One subclass of these antitumor agents, the 6-aziridinyl derivatives, act very much like mitomycin C and first need to be reduced before alkylating and cleaving DNA. Other subclasses, either the 6-acetamido quinone derivatives (6-acetamido-7-methyl-2,3-dihydro-1 H-pyrrolo [1,2-a]benzimidazole-5,8-diones (APBIs)) and the iminoquinone derivatives (6-acetamido-5-imino-7-methyl-2,3-dihydro-1H-pyrrolo [1,2-a]benzimidazole-8-ones (imino-APBIs)), need to be in an oxidized form to be cytotoxic (Skibo EB, Curr. Med. Chem. 3:47-78, 1996).

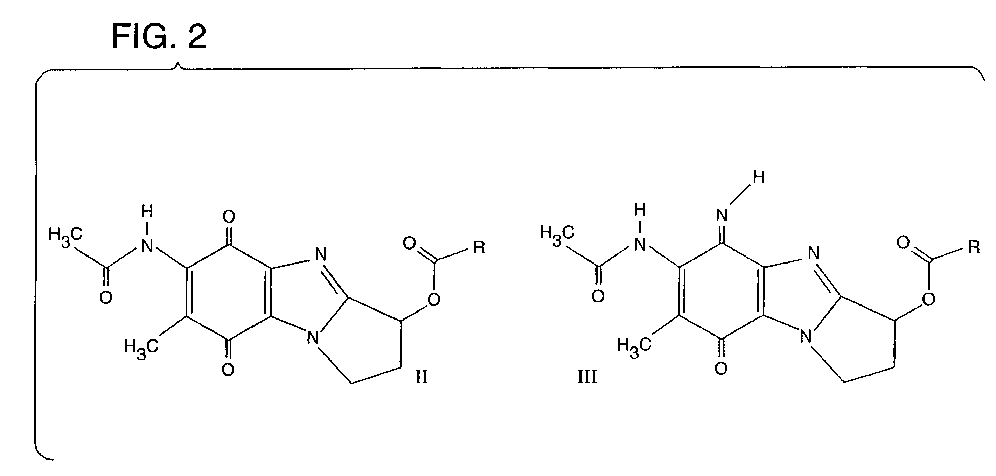

The structure of a APBI (II) and an imino-APBI (III) is provided in Figure 2. The mechanism of action of these compounds is different from that of mitomycin C. First, the compounds must be converted to their oxidized forms to be active. A recent study has reported a high inverse correlation between the level of cytotoxicity and the level of DT-diaphorase in cancer cells. DT-diaphorase is a 2-electron reducing enzyme which converts quinones to hydroquinones. Once in their oxidized forms, APBIs and imino-APBIs act as DNA intercalating agents and inhibit topoisomerase II activity.

A typical disadvantage of treatment with APBIs or imino-APBIs is that they are not very effective in cancer chemotherapy because cancer cells readily convert the oxidized form into the reduced form.

The present invention combines the insight that mitomycin is inactivated by oxidation within resistant cells with the fact that other cytotoxic compounds such as the APBIs can be activated by this same process. Bacterial and other cells have evolved the ability to detoxify mitomycin and other related compounds by the presence of (and especially by the genetic induction of) oxidizing enzymes (Johnson et al. 1997). Accordingly, the present invention discloses synergistic antibiotic compositions comprising at least two compounds where the first compound is selected from the group of compounds requiring chemical reduction in order to be cytotoxic and the second compound is selected from the group of compounds requiring chemical oxidation to become cytotoxic. For example, within a pharmaceutical composition comprising mitomycin C and/or an analog and APBIs or imino-APBIs, the antibacterial agents can act synergistically within the bacteria so that both antimicrobial agents together are more effective than if they were administered separately. Conditions that inactivate mitomycin C increase the activity of APBIs by converting reduced APBIs to activated, oxidized forms. Such conditions may involve the induction of proteins, such as MRCA. Since both mitomycin C and APBI have structural similarities, MRCA is expected to act on both compounds.

Thus, the present invention serves as a novel antibiotic strategy that minimizes the likelihood that bacterial strains will develop resistance to the antibiotic combination. Additionally, the present invention serves as a novel strategy to combat super-infection. Furthermore, the cytotoxicity of the antimicrobial combination may be decreased in comparison with that of mitomycin C or APBIs when each is administered separately because the synergistic effect of both compounds allows less of each compound to be administered to the patient.

### SUMMARY OF THE INVENTION

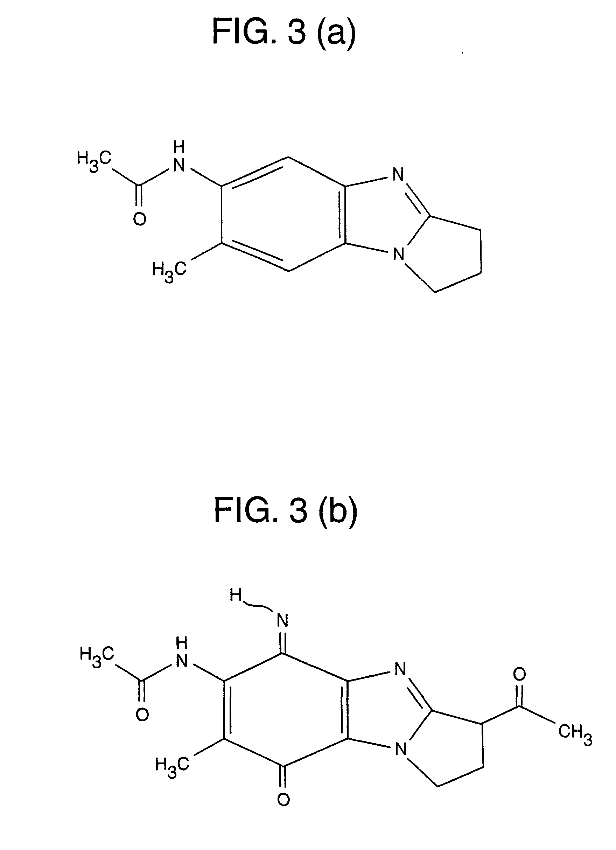

Accordingly, the present invention is an antimicrobial composition comprising a first antimicrobial agent and a second antimicrobial agent. The first antimicrobial agent can exist in either a reduced or oxidized form but is substantially more effective in its reduced form than in its oxidized form; and the second antimicrobial agent can also exist in either a reduced or oxidized form but is substantially more effective in its oxidized form than in its reduced form. Preferably, the first antimicrobial agent is a reductively activatable quinone compound, such as a mitomycin, preferably mitomycin C. Preferably, the second antimicrobial agent is an oxidatively activatable pyrrolo[1,2-]benzimidazole compound, such as 6-acetamido-7-methyl-2,3-dihydro-1H-pyrrolo [1,2-a]benzimidazole-5,8-dione-3-acetate, where the 3-acetate may be substituted with hydrogen or carbamate (Figure 3 (a)). In another embodiment, the oxidatively activatable pyrrolo[1,2-]benzimidazole compound is 6-acetamido-5-imino-7-methyl-2,3-dihydro-1H-pyrrolo [1,2-a]benzimidazole-8-one-3-acetate, where the 3-acetate may be substituted with hydrogen or carbamate (Figure 3(b)).

A further embodiment of the present invention is a pharmaceutical composition for treating a microbial disease which further comprises an antimicrobial composition and a pharmaceutically acceptable carrier. The antimicrobial composition itself is comprised of a first antimicrobial agent and a second antimicrobial agent. The first antimicrobial agent can exist in either a reduced or oxidized form but is substantially more effective in its reduced form than in its oxidized form and the second antimicrobial agent can also exist in either a reduced or oxidized form but is substantially more effective in its oxidized form than in its reduced form. The combination of both compounds in the composition increases the potency of both and thereby allows an effective lesser treatment dosage of each individual compound. The antimicrobial composition is present in an amount sufficient to effectively treat the microbial disease.

The embodiments of the present invention are used against bacterial pneumonia, salmonellosis, bacterial meningitis or other CNS infection, endocarditis, osteomyelitis, urinary tract infection, toxic shock syndrome, pharyngitis, bacterial endometriosis, diphtheria, septicemia, gastroenteritis, urinary tract infections, otitis media, salmonellosis, shigellosis, tuberculosis, staphylodermatitis, keratitis, impetigo, cellulitis, erysipelas or endophthalmitis.

Also disclosed is a method for treating a patient with a microbial disease, comprising administering the embodiments of the present invention discussed above, so as to elicit a therapeutic effect in the patient after administration of the pharmaceutical composition.

### BRIEF DESCRIPTION OF THE DRAWINGS

Figure 1 illustrates the chemical structure of mitomycin C (I).
Figure 2 illustrates the chemical structure of a APBI (II) and an imino-APBI (III).
Figure 3 shows the chemical structures of 6-acetamido-7-methyl-2,3-dihydro-1H-pyrrolo [1,2-a]benzimidazole-5,8-dione-3-acetate (a) and 6-acetamido-5-imino-7-methyl-2,3-dihydro-1H-pyrrolo [1,2-a]benzimidazole-8-one-3-acetate (b).

### DETAILED DESCRIPTION OF A PREFERRED EMBODIMENT OF THE INVENTION

The invention involves an antimicrobial composition comprising a first antimicrobial agent can exist in either a reduced or oxidized form but is substantially more effective in its reduced form than in its oxidized form and a second antimicrobial agent can exist in either a reduced or oxidized form but, is substantially more effective in its oxidized form than in its reduced form. In this manner, at least one component of the antimicrobial composition remains effective to combat microbial infection irrespective of whether the microorganisms have induced cellular resistance mechanisms to render one of the agents ineffective by oxidation or reduction mechanisms. Since the mechanism of inactivation of the first antimicrobial agent is substantially the same as the method of activation of the second antimicrobial agent, at any time at least one of the antimicrobial agents will be effective.

In addition to combating microbial resistance to antibiotics, the present invention is useful to combat the phenomenon of super-infection where a secondary infection occurs while a patient is being treated with an antibiotic for a first infection.

In particular, the present invention comprises a novel antimicrobial composition comprising a mitomycin and/or derivative or analog and APBI or imino-APBI. It is well known that mitomycin C alone can act as an antimicrobial agent. To be effective, mitomycin C must be in a reduced form. It is well known that APBI is an effective antitumor agent and is less cytotoxic than mitomycin C. However, to date APBI has not been used as an antimicrobial agent. We have discovered that APBI is effective as an antimicrobial agent in its oxidized form. APBI is not substantially effective as an antimicrobial agent once it is reduced. Alternatively, imino-APBI can be substituted for APBI.

In the present invention, the cellular mechanism that renders mitomycins ineffective, i.e., a mechanism that causes the re-oxidation of reduced mitomycin C, will enhance the conversion of APBI to the oxidized, active form. Therefore, a combination of both mitomycin and APBI is more effective and has a longer lasting effect as an antimicrobial composition than does either antimicrobial agent alone.

Microorganisms that have not become resistant to mitomycin C, i.e., which can still reduce mitomycin C, will be susceptible to mitomycin C. However, those microorganisms that have become resistant to mitomycin C as a result of cellular mechanisms that cause the re-oxidation of mitomycin C, for example, by the expression of MRCA (Johnson D.A., et al., J. Am. Chem. Soc. 119:2576-77, 1997), are susceptible to the antimicrobial action of APBI because MRCA converts APBI to its oxidized form. Thus, the antimicrobial compositions of the present invention can target both mitomycin C-resistant cells and cells that have not developed mitomycin resistance.

Thus, therapeutic compositions comprising the antimicrobial composition described above can be employed to treat patients suffering from a variety of microbial infections, such as pneumonia, bacterial meningitis or other CNS infection, endocarditis, peritonitis, empyema, staphylodermatitis, osteomyelitis, urinary tract infection, toxic shock syndrome, pharyngitis, bacterial endometriosis, impetigo, cellulitis, erysipelas, diphtheria, septicemia, gastroenteritis, urinary tract infections, otitis media, keratitis, endophthalmitis, salmonellosis, shigellosis and tuberculosis.

### Definitions:

As defined herein, a "reductively activatable quinone compound" is a compound which is activated by the reduction of the quinone, the elimination of one or more leaving groups and the formation of an alkylating quinone methide species. Once activated, the compounds are effective antimicrobial agents. Examples of reductively activatable quinone compounds include mitomycin C, porfiromycin, mitomycin A and mitomycin B and their corresponding mitosene analogs and derivatives and the 6-aziridinyl pyrrolo[1,2-]benzimidazoles. Also having similar activity are the following: anthracyclines, such as daunorubicin and adriamycin, streptonigrin, carminic acid, and peptide antibiotics such as neocarzinostatin, bleomycin and tallysomycin, and analogs and derivatives thereof.

Mitomycin C is a preferred reductively activatable quinone compound. Other preferred reductively activatable quinone compounds are derivatives of mitomycin C which contain the same ring structure that mitomycin has but has different substituents.

An "oxidatively activatable pyrrolo[1,2-]benzimidazole compound" is a compound which is activated by oxidation and has the ring structure for the quinone form (II) or the ring structure for the iminoquinone form (III). Once activated, the compounds are effective antimicrobial agents. The 6-acetamido substituent can be varied extensively, so long as the NHCO remains in a relatively flat molecular plain, which allows intercalation of the molecule.

The structures of two oxidatively activatable pyrrolo[1,2-]benzimidazole compounds are shown as (II) and (III) in Figure 2. The Z substituent can include moieties such as acetate, carbamate, hydride, hydroxyl, propionate, benzoate, valerate, and methoxyacetate moieties (Schulz WG, et al., J. Med. Chem. 38:109-18, 1995). A complete list of acids for use in producing the Z substituent is provided in U.S. Patent No. 5,246,955 (Col. 21, line 42 to Col. 22, line 43)

The term "antimicrobial agent" means at least one antimicrobial agent which is effective against a variety of microorganisms. The phrase "substantially more effective in its reduced form than its oxidized form" means that the reduced form is at least 10 times more effective as an antimicrobial agent than its oxidized form. The phrase "substantially more effective in its oxidized form than its reduced form" means that the oxidized form is at least 10 times more effective as an antimicrobial agent than its reduced form.

"Treating a patient with a microbial disease" means administering the composition to humans or other mammals which have a microbial disease. Microbial diseases include but are not limited to pneumonia, bacterial meningitis and other CNS infections, endocarditis, peritonitis, empyema, staphylodermatitis, osteomyelitis, urinary tract infection, toxic shock syndrome, pharyngitis, bacterial endometriosis, impetigo, cellulitis, erysipelas, diphtheria, septicemia due to any cause, gastroenteritis, urinary tract infections, otitis media, keratitis, endophthalmitis, salmonellosis, shigellosis and tuberculosis.

### Preparation of the Antimicrobial Compositions

### A. Syntheses of ABPI and Imino-ABPI Derivatives, Syntheses of Mitomycin C and its Reductively Activatable Analogs.

Synthetic methods for the preparation of ABPI and imino-ABPI derivatives are provided in U.S. Patent Nos. 5,015,742 and 5,246,955.

Synthetic methods for the preparation of mitomycin C and its derivatives and references cited therein summarized by WA Remers (Ch. 3 "Mitomycin C and analog Development." In: MITOMYCIN C: CURRENT STATUS AND NEW DEVELOPMENTS, Ed. by SK Carter and ST Crooke, Academic Press, New York City, 1979, pp. 27-32). Synthetic methods for the preparation of mitomycin C and its derivatives are disclosed in Jimenez, et al., U.S. Patent No. 5,523,411, where a simple, four-step method is claimed for the synthesis of mitomycin C; and in the various patents of Remers, et al., U.S. Patent No. 4,746,746, where a multiplicity of mitomycin derivatives with a large number of substitutions are claimed as well as U.S. Patent Nos. 4,460,599; 4,888,341; and 5,098,926 describe the methodology for synthesizing 6-substituted mitomycin analogs. The patent of Kaneko, T., et al., U.S. Patent No. 5,374,739 describes the synthesis of mitomycin C analogs in which the 7-amino group bears a 5-membered heterocyclic substituent. The synthesis of bis-amidine analogs of mitomycin C are described in Vyas, D., et al., U.S. Patent Nos. 4,579,737 and 4,590,074. Mitomycin C derivatives produced by reacting p-phenyl or p-phenylenediamine with mitomycin C and which exhibit good antibacterial activity is described in Nakano, K., et al., U.S. Patent No. 4,231,936. Most recently, 2,7-diaminomitosene analogues are disclosed in Peterson, D, U.S. Patent No. 5,629,427.

### B. Specific Antimicrobial Compositions

Specific antimicrobial compositions for use in the invention include a wide range of combinations of mitomycin compounds and APBI compounds. The compositions may contain about 5 to 95% by weight mitomycins and about 95 to 5% by weight APBIs. More preferably, the compositions will contain 40 to 60% of each. In general, a larger percentage of APBI will be chosen in cases where the microorganisms are known (having been isolated and cultured in a lab) to be mitomycin resistant. The small amounts of mitomycin present in this case will continue to induce cells to support their production of the oxidative enzyme. Conversely, when the cells are known or suspected to be mitomycin sensitive, higher percentage amounts of mitomycin will be employed to achieve the most efficient attack on the infecting microorganisms.

Much treatment of human infections is undertaken empirically. Patients initially treated with a composition of this invention having a higher percentage of mitomycin may be switched to a higher percentage of APBI later, if the infection is found to be unresponsive to the initial treatment.

In one aspect, the inventive composition comprises a single reductively activatable quinone compound and a single oxidatively activatable pyrrolo[1,2] benzimidazole compound. In other aspects, it may be advantageous to combine one or more reductively activatable quinone compounds with one or more oxidatively activatable pyrrolo[1,2-]benzimidazole compounds. For example, an antimicrobial composition may comprise mitomycin C, 7-N-(p-aminophenyl)-mitomycin C, APBI-acetate and imino-APBI-benzoate.

Susceptibility testing in order to determine to which antibiotic an organism will respond is routinely practiced in hospital and other microbiology laboratories. It is well known in the art and a medically accepted practice. The present invention envisions that such testing with various combinations of synergistic compounds, such as described herein, will be routinely performed to determine the optimum antimicrobial combination.

### Administration of the Therapeutic Composition

The antimicrobial composition of the present invention can be used in therapeutic compositions for the treatment of a variety of gram-negative or gram-positive bacterial infections.

The antimicrobial composition shows in vitro activity against various microorganisms, including Klebsiella pneumoniae, Bacillus cereus, Mycobacterium smegmatis, Escherichia coli, Salmonella gallinarum, Staphylococcus aureus, Pseudomonas aeruginosa, Streptococcus sp. beta hemolyticus, Streptococcus aureus, Bacillus subtilis, Sarcina lutea, Salmonella typhi, Shigella flexneri, Mycobacterium tuberculosis, Streptococcus haemolyticus, Enterococcus faecalis, Diplococcus pneumoniae, Corynebacterium diphtheriae, Serratia marcescens, Pseudomonas cepacia, Proteus vulgaris, Shigella sonnei and others.

As a consequence, a therapeutic composition comprising the antibacterial composition can be used to combat a variety of microbial infections, such as pneumonia, bacterial meningitis and other CNS infections, endocarditis, peritonitis, empyema, staphylodermatitis, osteomyelitis, urinary tract infection, toxic shock syndrome, pharyngitis, bacterial endometriosis, impetigo, cellulitis, erysipelas, diphtheria, septicemia, gastroenteritis, urinary tract infections, otitis media, keratitis, endophthalmitis, salmonellosis, shigellosis and tuberculosis.

The therapeutic compositions are preferably presented for administration to patients as powders, tablets, capsules, aerosols, topical creams, parenteral solutions or suspensions, non-parenteral solutions or suspensions, oral solutions or suspensions and the like.

Powders are prepared by mixing the antimicrobial composition with a diluent, such as starch, lactose and the like, or a base, such as kaolin, dicalcium phosphate, and the like. Tablets are made by preparing the same powder mixture, adding a lubricant, such as corn syrup, gelatin solution, methylcellulose solution, and the like, and pressing into tablet form. When desired, the tablet can be provided with a protective coating, such as a coating of sugar and methylcellulose. Capsules are prepared by using the same powder mixture and filling the mixture into formed gelatin sheaths. Lubricants such as talc, magnesium stearate, calcium stearate and the like can be used.

Aerosols are prepared by packaging the antimicrobial composition in a pressurized aerosol container together with a gaseous or liquefied propellant such as dicholorodifluoromethane, carbon dioxide, nitrogen and the like.

Because the present invention is most effective as a last treatment resort for patients who have systemic infections which are or are becoming resistant to Vancomycin treatment, the preferred embodiment encompasses the intravenous (I.V.) route of administration. For administering the antibacterial composition in solution or suspension, the antibacterial composition is combined with a buffering solution, or a preservative. Additionally, the antibacterial composition can be combined with a surfactant or wetting agent to facilitate uniform distribution of the composition.

Other embodiments of the therapeutic compositions can be administered from separate parenteral solution containers and the rate of administration from each varied with respect to the regimen described above. Such a parenteral regimen will be particularly suitable for bacterial meningitis and other CNS infections, endocarditis, peritonitis, empyema, osteomyelitis, toxic shock syndrome, pharyngitis, bacterial endometriosis, cellulitis, diphtheria, septicemia, gastroenteritis, endophthalmitis, salmonellosis, shigellosis and tuberculosis.

For purposes of preparing a topical cream, the antibacterial composition of the present invention can be combined with one or more lipids to enhance transport of the composition through membranes. Topical creams are useful for the treatment of bacterial infections of the skin, such as staphylodermatitis, impetigo, cellulitis, and erysipelas.

Generally, about 0.01 to 100 mg/kg body weight, more preferably 1 to 50 mg/kg body weight of the antimicrobial composition is administered to the patient during treatment. Routine antibiotic susceptibility testing, well known in the art, may be used to determine the optimal combination of the components for the particular infection or empirical methods as described above may be used.

Preferably, both first and second antimicrobial agents will be administered at substantially the same time. This means that the powders, capsules, solutions or suspensions, topical creams, and tablets will contain both first and second antimicrobial agents; and the patient will be treated with the agents at the same time.

However, in some cases it may be advantageous to first administer one of the antimicrobial agents first and then the other second. In these cases, it is preferable to administer the first antimicrobial agent first, i.e., preferably the reductively activatable mitosene compound and more preferably mitomycin C. That way mitomycin C induces expression of MRCA which can oxidatively activate the second antimicrobial agent, ABPI. As described above, the second antimicrobial agent is preferably a oxidatively activated azamitosene compound and more preferably ABPI. In one embodiment, ABPI is administered about 8 to 24 hours after administering mitomycin C. This assures that those cellular mechanisms which confer mitomycin resistance are activated and effectively convert ABPI into the oxidatively activated form.

### EXAMPLES

Methods for *in vitro* and *in vivo* testing of antibiotics are well known in the art. Even for binary compositions of antibiotics, such testing is routine. See, for example, the following references and the methodologies cited therein: Barrett MS et al., Diagn. Microbiol. Infect. Dis. 25:147-49, 1996; Finch RG, Drugs 1996 51 (Suppl 1): 31-37, 1996; Jones RN, Diagn. Microbiol. Infect. Dis. 26:99-102, 1996.

In *in vitro* antibiotic sensitivity tests, compositions comprising various amounts of a) mitomycin C alone, b) ABPI alone and c) variable combinations of mitomycin C and ABPI are administered to a variety of bacteria strains which are resistant to mitomycin C. The efficacy of the different compositions on the tested bacterial strains is compared. Besides minimum inhibitory concentrations (MIC), the antibiotics can be tested in time-kill studies, both of which are disclosed in Spangler SK, et al., Antimicrob. Ag. Chemoth. 41(1):148-55, 1997

For *in vivo* testing of the antibiotic compositions, groups of rodents or other suitable test animals are initially infected with bacteria and then treated with a range of combination compositions, expected on the basis of in vitro testing to be efficacious. The animals are then compared in their survival to untreated, but infected, control animals. The most effective composition for use in vivo can thus be determined. At post mortem, the treated animals may be examined for any signs of drug toxicity.

In another *in vivo* test, rodents are infected with a bacteria resistant to mitosene antibiotics and given the combination antibiotic. The rodents are then observed for signs of recovery.

### Parenteral Product

A sterile aqueous suspension for parenteral injection, containing in 1 ml, 30 mg of an active ingredient for treating a infectious disease, is prepared from the following types and amounts of ingredients:

| Ingredient | Amount |
|---|---|
| Active Ingredients (micronized) | 30 g |
| Polysorbate 80 | 5 g |
| Methylparaben | 2.5 g |
| Propylparaben | 0.17 g |
| Water for injection q.s. | 1000 mL |

All the ingredients, except the active ingredient, are dissolved in the water and the solution sterilized by filtration. To the sterile solution is added the sterilized active ingredient, finely divided by means of an air micronizer, and the final suspension is filled into sterile vials and the vials sealed.

The composition so prepared is useful for treating a infectious disease at a dose of milliliter (1 mL) three times a day.

### Intravenous Product

| Ingredients | Amount |
|---|---|
| Active Ingredient | 15 mg |
| Mannitol | 100 mg |
| Tris(hydroxymethyl)aminomethane | 24.2 mg |
| HCl | q.s. |
| Water for injection | 9.9 mL |

All the ingredients are supplied as a lyophilized powder in a sterile vial. 9.9 mL of sterile water for injection is added and the mixture is shaken until the components are thoroughly dissolved. The final pH of the solution is in the range of 7.8-8.1 but the drug can be administered in a greater range of about pH 6-8.5. The entire contents are injected into the patient for treatment of an infectious disease.

The dose ranges for the two compounds can vary depending on the severity of the infection, the route of administration, the age of the patient and the formulation form of the two compounds in the composition.

## Claims

1. An antimicrobial composition comprising
a. a first antimicrobial agent and
b. a second antimicrobial agent,
said first antimicrobial agent being substantially more effective in its reduced form than in its oxidized form and said second antimicrobial agent being substantially more effective in its oxidized form than in its reduced form.

2. The antimicrobial composition of claim 1, wherein the first antimicrobial agent is a reductively activatable quinone compound and/or the second antimicrobial agent is an oxidatively activatable pyrrolo[1,2]benzimidazole compound.

3. The antimicrobial composition of claim 2, wherein the reductively activatable quinone compound is selected from the group consisting of mitomycin C, porfiromycin, mitomycin A and mitomycin B and their corresponding mitosene compounds; or anthracyclines, such as daunorubicin and adriamycin, streptonigrin, carminic acid; or peptide antibiotics such as neocarzinostatin, bleomycin and tallysomycin; or analogs and derivatives thereof, preferably the reductively activatable quinone compound is mitomycin C.

4. The antimicrobial composition of claim 2, wherein the oxidatively activatable pyrrolo[1,2-]benzimidazole compound has the structural formula: 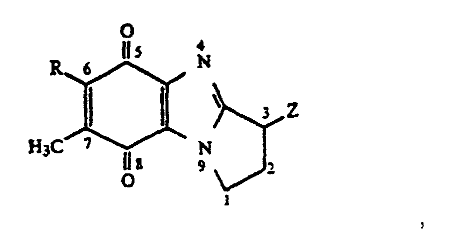 wherein Z is selected from the group consisting of H, acetate or carbamate; or the oxidatively activatable pyrrolo[1,2-]benzimidazole compound has the structural formula: 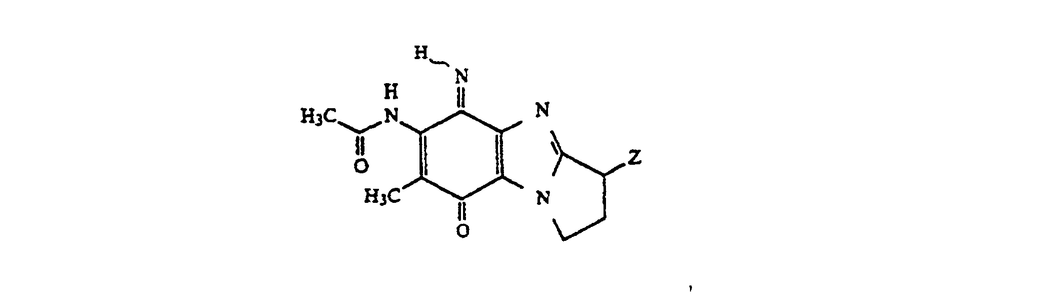 wherein Z is selected from the group consisting of H, acetate or carbamate.

5. The antimicrobial composition of claim 4, wherein the oxidatively activatable pyrrolo[1,2-]benzimidazole compound is 6-acetamido-7-methyl-2,3-dihydro-1H-pyrrolo [1,2-a]benzimidazole-5,8-dione-3-acetate and/or 6-acetamido-5-imino-7-methyl-2,3-dihydro-1-H-pyrrolo[1,2-a]benzimidazole-8-one-3-acetate.

6. A pharmaceutical composition for treating a microbial disease comprising
a. an antimicrobial composition according to any of claims 1 to 5 in an amount sufficient to render it effective to treat the microbial disease, and
b. a pharmaceutically acceptable carrier.

7. The pharmaceutical composition according to 6, wherein the microbial disease is bacterial pneumonia, salmonellosis, bacterial meningitis or other CNS infection, endocarditis, osteomyelitis, urinary tract infection, toxic shock syndrome, pharyngitis, bacterial endometriosis, diphtheria, septicemia, gastroenteritis, urinary tract infections, otitis media, salmonellosis, shigellosis, tuberculosis, staphylodermatitis, keratitis, impetigo, cellulitis, erysipelas or endophthalmitis.

8. The pharmaceutical composition according to any of claims 6 or 7, wherein the pharmaceutical composition is administered to the patient by oral, intravenous or intraperitoneal administration means or as a topical cream.

## Patentansprüche

1. Antimikrobische Zusammensetzung, umfassend
a) ein erstes antimikrobisches Mittel, und
b) ein zweites antimikrobisches Mittel,
wobei das erste antimikrobische Mittel im wesentlichen wirksamer in seiner reduzierten Form als in seiner oxidierten Form ist, und das zweite antimikrobische Mittel im wesentlichen wirksamer in seiner oxidierten Form als in seiner reduzierten Form ist.

2. Antimikrobische Zusammensetzung nach Anspruch 1, wobei das erste antimikrobische Mittel eine reduktiv aktivierbare Quinon-Verbindung und/oder das zweite antimikrobische Mittel eine oxidativ aktivierbare Pyrrolo[1,2]benzimidazol-Verbindung ist.

3. Antimikrobische Zusammensetzung nach Anspruch 2, wobei die reduktiv aktivierbare Quinon-Verbindung ausgewählt ist aus der Gruppe aus Mitomycin C, Porfiromycin, Mitomycin A und Mitomycin B und deren entsprechenden Mytosene-Verbindungen; oder Anthracycline, wie beispielsweise Daunorubicin und Adriamycin, Streptonigrin, Carminsäure; oder Peptid-Antibiotika, wie beispielsweise Neocarzinostatin, Bleomycin und Tallysomycin; oder Analoga und Derivate davon, wobei die reduktiv aktivierbare Quinon-Verbindung vorzugsweise Mitomycin C ist.

4. Antimikrobische Zusammensetzung nach Anspruch 2, wobei die oxidativ aktivierbare Pyrrolo[1,2]benzimidazol-Verbindung die Strukturformel: 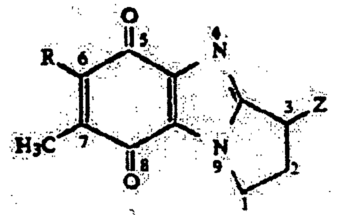 besitzt, wobei Z ausgewählt ist aus der Gruppe aus H, Acetat oder Carbamat; oder wobei die oxidativ aktivierbare Pyrrolo[1,2]benzimidazol-Verbindung die Strukturformel: 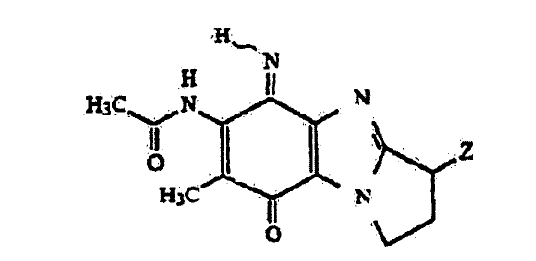 besitzt, wobei Z ausgewählt ist aus der Gruppe aus H, Acetat oder Carbamat.

5. Antimikrobische Zusammensetzung nach Anspruch 4, wobei die oxidativ aktivierbare Pyrrolo[1,2]benzimidazol-Verbindung ein 6-Acetamido-7-methyl-2,3-dihydro-1Hpyrrolo[1,2-a]benzimidazol-5,8-dion-3-acetat und/ oder ein 6-Acetamido-5-imino-7-methyl-2,3-dihydro-1-H-pyrrolo[1,2-a]benzimidazol-8-on-3-acetat ist.

6. Pharmazeutische Zusammensetzung für die Behandlung einer mikrobischen Krankheit, umfassend
a) eine antimikrobische Zusammensetzung nach einem der Ansprüche 1 bis 5, in einer Menge, die ausreicht, um sie zur Behandlung der mikrobischen Krankheit wirksam zu machen, und
b) einen pharmazeutisch akzeptablen Carrier.

7. Pharmazeutische Zusammensetzung nach Anspruch 6, wobei die mikrobische Krankheit entweder bakterielle Pneumonie, Salmonellose, bakterielle Meningitis oder eine andere CNS-Infektion, Endocarditis, Osteomyelitis, Harnwegsinfektion, toxisches Schocksyndrom, Pharyngitis, bakterielle Endometriose, Diphtherie, Septikhämie, Gastroenteritis, Harnwegsinfektionen, Mittelohrentzündung, Salmonellose, Shigellose, Tuberkulose, Staphylokokkendermatitis, Keratitis, Impetigo, Cellulitis, Erysipel oder Endophthalmitis ist.

8. Pharmazeutische Zusammensetzung nach einem der Ansprüche 6 oder 7, wobei die pharmazeutische Zusammensetzung einem Patienten durch orale, intravenöse oder intraperitoneale Verabreichungsmittel oder als topische Creme verabreicht wird.

## Revendications

1. Composition antimicrobienne comprenant
a. un premier agent antimicrobien et
b. un deuxième agent antimicrobien
ledit premier agent antimicrobien étant considérablement plus efficace dans sa forme réduite que dans sa forme oxydée, et ledit deuxième agent antimicrobien étant considérablement plus efficace dans sa forme oxydée que dans sa forme réduite.

2. La composition antimicrobienne de la revendication 1, dans laquelle le premier agent antimicrobien est un composé de quinone activable par réduction et/ou le deuxième agent antimicrobien est un composé de pyrrolo[l,2]benzimidazole activable par oxidation.

3. La composition antimicrobienne de la revendication 2, dans laquelle le composé de quinone activable par réduction est sélectionné parmi le groupe de mitomycine C, porfiromycine, mitomycine A et mitomycine B et leurs composés de mitosène correspondants; ou parmi les anthracyclines tels que la daunorubicine et l'adriamycine, la streptonigrine et l'acide carminique; ou parmi les antibiotiques peptidiques tels que le néocarzinostatine, la bléomycine et la tallysomycine; ou les analogues et dérivés de ces dernières, la mitomycine C étant le composé de quinone activable par réduction préféré.

4. La composition antimicrobienne de la revendication 2, dans laquelle le composé de pyrrolo[1,2-]benzimidazole activable par oxidation, a la formule structurale: 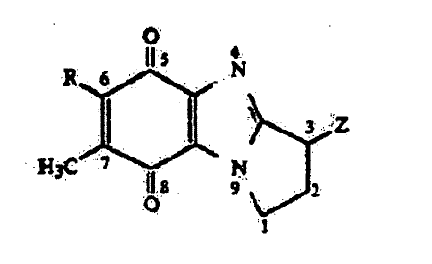 dans laquelle Z est sélectionné parmi le groupe consistant de H, acétate ou carbamate; ou le composé de pyrrolo[1,2-]benzimidazole activable par oxidation a la formule structurale: 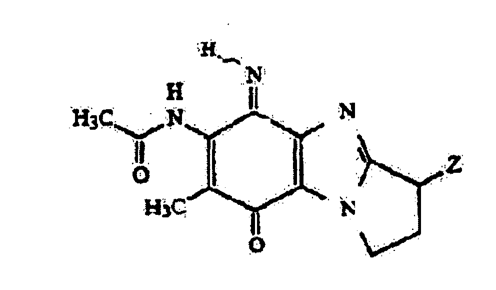 dans laquelle Z est sélectionné parmi le groupe consistant de H, acétate ou carbamate;

5. La composition antimicrobienne de la revendication 2, dans laquelle le composé de pyrrolo[1,2-]benzimidazole activable par oxidation est le 6-acétamido-7-méthyle-2,3-dihydro-lH-pyrrolo[1,2-a]benzimidazole-5,8-dione-3-acétate et/ou le 6-acétamido-5-imino-7-méthyle-2,3-dihydro-1-H-pyrrolo[1,2-a]benzimidazole-8-one-3-acétate.

6. Une composition pharmaceutique pour le traitement de maladies microbiennes comprenant
a. une composition antimicrobienne selon l'une quelconque des revendication 1 à 5, dans une quantité suffisante pour assurer l'efficacité du traitement de la maladie microbienne, et
b. un support acceptable en pharmacie.

7. La composition pharmaceutique selon la revendication 6, dans laquelle la maladie microbienne est la pneumonie bactérienne, la salmonellose, la méningite bactérienne ou d'autres infections du système nerveux central, l'endocardite, l'ostéomyélite, l'infection du tract urinaire, le syndrome de choc toxique, la pharyngite, l'endométriose bactérienne, la diphthérie, la septicémie, la gastroentérite, les infections du tract urinaire, l'otite moyenne, la salmonellose, la shigellose, la tuberculose, la dermatite staphylococcique, la kératite, l'impétigo, la cellulite, l'érysipèle ou l'endophthalmite.

8. La composition pharmaceutique selon l'une quelconque des revendication 6 ou 7, ladite composition pharmaceutique étant administrée au patient par voie orale, intravéneuse ou intrapéritonéale ou topiquement sous forme de crème.
